Europäisches Patentamt

European Patent Office

Office européen des brevets

(19)

(11) Numéro de publication : **0 436 650 B1**

(12)

# FASCICULE DE BREVET EUROPEEN

(45) Date de publication du fascicule du brevet :
31.03.93 Bulletin 93/13

(51) Int. Cl.⁵ : **A61K 7/00,** A61K 9/127,
A61K 31/575

(21) Numéro de dépôt : **89911565.3**

(22) Date de dépôt : **02.10.89**

(86) Numéro de dépôt international :
**PCT/FR89/00507**

(87) Numéro de publication internationale :
**WO 90/03778 19.04.90 Gazette 90/09**

(54) PHASES LAMELLAIRES LIPIDIQUES HYDRATEES OU LIPOSOMES A BASE D'ECDYSTEROIDES.

(30) Priorité : **03.10.88 FR 8812909**

(43) Date de publication de la demande :
**17.07.91 Bulletin 91/29**

(45) Mention de la délivrance du brevet :
**31.03.93 Bulletin 93/13**

(84) Etats contractants désignés :
**AT BE CH DE GB IT LI LU NL SE**

(56) Documents cités :
**EP-A- 0 004 223
DE-A- 1 793 642**

(73) Titulaire : **LVMH RECHERCHE
48-50, rue de Seine, B.P. 79
F-92703 Colombes Cédex (FR)**

(72) Inventeur : **MEYBECK, Alain
Les Poissons 20 ter, rue de Bezons
F-92400 Courbevoie (FR)**
Inventeur : **BONTE, Frédéric
5, place Charras
F-92400 Courbevoie (FR)**

(74) Mandataire : **Portal, Gérard et al
Cabinet Beau de Loménie 55, rue
d'Amsterdam
F-75008 Paris (FR)**

EP 0 436 650 B1

## Description

La présente invention concerne essentiellement des compositions à base de phases lamellaires lipidiques hydratées ou de liposomes contenant un ecdystéroïde, de préférence l'ecdystérone, ou l'un de ses dérivés ; et des compositions cosmétiques, pharmaceutiques, notamment dermatologiques, de sériculture ou phytosanitaires l'incorporant.

Les ecdystéroïdes sont un groupe de 2,3,14-trihydroxy-Δ-7-6-kétostéroïdes. On peut citer l'α-ecdysone ou (2β,3β,14α-22[R], 25-pentahydroxy-7-cholestène-6-one) ; la 2-déoxyecdysone ou (3β,14β,22[R],25-tétrahydroxy-5β-7-cholestène-6-one), l'Ecdystérone ou β-ecdysone ou 2β,3β,14α,20β,22,25-hexahydroxy-7-cholestène-6-one ; la β-ecdysone-22-acétate ou 20-hydroxyecdysone-22-acétate ou 7-cholestène-2β,3β,14,20, 22[R],25-hexol-6-one-22-acétate ; la 5-hydroxyecdystérone ou 5β,7-cholestène-2β,3β,5α,14,20,22[R]25-heptahydroxy-6-one ; la β-2-déoxyecdysone ou 3β,14,20,22[R]25-pentapentahydroxy-5β,7-cholestène-6-one. Les ecdystéroïdes, et en particulier l'ecdystérone (dans certains cas appelée β-ecdysone ou encore crustecdysone), sont bien connus dans la littérature et cités dans le Merck Index, 10e édition, 1983, page 505, n° 3 470.

On sait que les ecdystéroïdes, et en particulier l'ecdystérone, jouent un rôle important aussi bien dansle règne animal chez les insectes que dans le règne végétal. Chez les insectes, ces hormones jouent un rôle clé dans la croissance et la reproduction. L'ecdystérone intervient en particulier dans les différentes métamorphoses jusqu'à la formation de l'insecte adulte (voir publication du CNRS : Biologie 1990, "Enjeux et Problématiques" de A. Berkaloff et al.)

Chez les plantes, l'activité de ces substances n'est pas totalement élucidée. Elles semblent agir sur la floraison (CIENCIA e Cultura (1980), volume 32, n° 10, pages 1384-1390).

Par ailleurs, on connaît déjà l'emploi de phases lamellaires lipidiques hydratées ou de liposomes dans des compositions cosmétiques, ou pharmaceutiques, notamment dermatologiques, dans lesquelles on incorpore divers principes actifs (FR-A-2 540 381).

Il a maintenant été découvert, de manière tout à fait surprenante et inattendue, que l'incorporation d'un ecdystéroïde, de préférence l'ecdystérone, ou un dérivé dudit ecdystéroïde, ou un extrait végétal ou animal contenant ledit ecdystéroïde ou dérivé d'ecdystéroïde, au moins en partie dans une phase lamellaire lipidique hydratée ou dans des liposomes, provoquait une activité exacerbée de cette substance ou de cet extrait. Ceci concerne en outre, et également de manière tout à fait inattendue, toutes les activités connues de l'ecdystéroïde précité ou d'un extrait contenant celui-ci. Une amélioration encore plus radicale d'activité a été observée en ce qui concerne l'activité de régénération de la structure cutanée et de l'activité anti-vieillissement.

En conséquence, un effet de synergie est obtenu par l'incorporation d'un ecdystéroïde, de préférence l'ecdystérone ou un dérivé d'ecdystéroïde ou un extrait végétal ou animal contenant ledit ecdystéroïde, au moins en partie dans des phases lamellaires lipidiques hydratées ou dans des liposomes.

Ainsi, la présente invention a pour but de résoudre le nouveau problème technique consistant en la fourniture d'une nouvelle formulation d'ecdystéroïde(s), de préférence l'ecdystérone ou un dérivé dudit ecdystéroïde, ou un extrait végétal ou animal contenant ledit ecdystéroïde, permettant de potentialiser son efficacité pour permettre son utilisation dans les compositions cosmétiques, pharmaceutiques, notamment dermatologiques, ainsi que dans les compositions pour la sériculture et les compositions phytosanitaires.

La présente invention résout, pour la première fois, ce nouveau problème technique de manière satisfaisante.

Ainsi, selon un premier aspect, la présente invention fournit une composition à base de phases lamellaires lipidiques hydratées ou de liposomes, caractérisée en ce que lesdites phases lamellaires lipidiques hydratées ou lesdits liposomes contiennent au moins en partie au moins un ecdystéroïde ou un dérivé d'ecdystéroïde, ou un extrait végétal ou animal contenant ledit ecdystéroïde ou un dérivé dudit ecdystéroïde.

Selon un mode de réalisation particulier, l'ecdystéroïde précité est l'ecdystérone, ou un dérivé d'ecdystérone, notamment un dérivé acylé, hydroxylé ou désoxylé de celle-ci.

Selon un mode particulièrement avantageux de l'invention, le dérivé de l'ecdystérone est choisi parmi le groupe consistant de la β-ecdysone-2-acétate, la β-ecdysone-3-acétate, la β-ecdysone-2,3-diacétate, la β-ecdysone-2,3,22-triacétate, la β-ecdysone-2,3,22,25 -tétraacétate; la 5-hydroxyecdystérone et la 2-déoxyecdystérone.

Selon une variante de réalisation, l'ecdystéroïde précité est l'β-ecdysone ou un de ses dérivés, notamment un acétate.

Selon une autre caractéristique particulière de l'invention, l'extrait précité animal ou végétal contenant l'ecdystéroïde, de préférence l'ecdystérone, est unextrait de Polypodium vulgare, Ajuga decumbens ou de Cyanotis arachnoïdea.

Selon une autre caractéristique particulière de l'invention, l'ecdystéroïde ou son dérivé est incorporé au

2

moins en partie dans la phase lipidique des phases lamellaires lipidiques hydratées ou des liposomes.

Dans la présente description et les revendications, le terme "lipidique" dans l'expression "phase lipidique" ou "phase lamellaire lipidique" couvre toutes les substances comprenant une chaîne carbonée dite grasse, généralement supérieure à 5 atomes de carbone.

Selon l'invention, on utilise des lipides amphiphiles, c'est-à-dire constitués de molécules possédant un groupe hydrophile indifféremment ionique ou non ionique et un groupe lipophile, ces lipides amphiphiles étant susceptibles de former des phases lamellaires lipidiques ou des liposomes en présence d'une phase aqueuse. En particulier, citons parmi ces lipides : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja, une phosphatidylsérine, une sphyngomyéline, un céramide, un cérébroside ou un stéarate de polyglycéroloxyéthyléné.

Selon un mode de réalisation avantageux de l'invention, la concentration de l'ecdystéroïde est comprise entre 0,001 et 30 % en poids de ladite phase lipidique et encore de préférence entre 0,01 et 10 % en poids de la phase lipidique.

La préparation des phases lamellaires lipidiques hydratées ou des liposomes contenant au moins en partie au moins un ecdystéroïde selon l'invention peut être réalisée selon l'un des procédés connus pour incorporer des substances actives, notamment des stéroïdes, dans les phases lamellaires lipidiques hydratées ou les liposomes.

Le procédé le plus simple est celui décrit par Bangham dans J. Mol. Biol. (1965) 13, p. 238-252, qui consiste à mettre les lipides en solution organique volatile puis à évaporer le solvant dans un ballon rotatif de manière à obtenir une pellicule mince de lipides à l'intérieur du ballon rotatif, puis on ajoute de l'eau dans le ballon rotatif, ce qui permet d'obtenir, sous agitation, une suspension de liposomes. Un perfectionnement à ce procédé consiste à utiliser des ultrasons pour homogénéiser les liposomes obtenus.

Selon un mode de réalisation préféré, selon la présente invention, on utilise un procédé d'atomisation des constituants de la phase lipidique, cette atomisation étant suivie d'une dispersion en milieu aqueux, puis éventuellement d'une homogénéisation sous pression, conformément aux procédés décrits dans EP-B1-0087 993 et FR-A-2 534 487 ou EP-B1-107 559.

Selon un mode de réalisation préféré de l'invention, l'ecdystéroïde ou son dérivé ou un extrait végétal ou animal en contenant est incorporé dans la phase lipidique. On dissout ainsi l'ecdystéroïde ou son dérivé, ou un extrait le contenant, avec les constituants de la phase lipidique, avant atomisation, dans une solution organique contenant au moins un lipide amphiphile, tel que la lécithine de soja, et éventuellement un composé hydrophobe lipophile tel que le cholestérol ou le β-sitostérol. De préférence, le solvant est choisi parmi le dichlorométhane, le chloroforme ou le méthanol, ou l'un de leurs mélanges.

La solution organique peut avantageusement contenir un agent anti-oxydant tel que l'α-tocophérol.

La poudre lipidique obtenue est dispersée dans un milieu aqueux convenable, par exemple une solution tampon PBS, une solution de glucose ou une solution de chlorure de sodium.

On obtient ainsi des phases lamellaires lipidiques peu hydratées ou une solution de liposomes selon que l'on a choisi de disperser la poudre lipidique dans relativement peu, c'est-à-dire d'environ 50 à environ 90 %, ou beaucoup, c'est-à-dire plus de 90 %, de milieu aqueux, ainsi qu'il est exposé dans le document précité (EP-B1-0087 993).

Selon un mode de réalisation avantageux, surtout dans le cas d'une composition de liposomes, après avoir éventuellement homogénéisé la composition obtenue, les compositions de phases lamellaires lipidiques hydratées ou de liposomes sont gélifiées par mélange avec un gel, tel qu'un gel de polymére vinylique, en particulier commercialisé sous la dénomination commerciale Carbopol® 940. Cette procédure de gélification est également décrite dans EP-B1-0087 993, en particulier dans les exemples.

Les ecdystéroïdes ou leurs dérivés, de préférence l'ecdystérone ou ses dérivés, sont obtenus sous forme isolée, ou sous forme d'un extrait à partir de toutes sources naturelles disponibles, ou encore par un procédé de synthèse chimique. Les principales sources naturelles d'ecdystéroïdes sont les insectes et surtout un grand nombre de plantes. Egalement, un certain nombre de procédés de synthèse ont été mis au point.

## Extraction à partir d'insectes

La quantité d'ecdystéroïde, en particulier d'α ou β-ecdysone, présente dans les insectes est extrêmement faible. Chez le papillon Bombyx mori, elle est par exemple de $5.10^{-6}$ % en poids d'insecte (voir A. Butenandt et al., Z. Naturforsch. B., 9, 389, (1954)).

L'extraction d'ecdysteroïde a partir d'insectes ne peut donc généralement pas constituer un procédé d'obtention industriel.

Cependant, dans le document Hunger-Ricci CH-A-478 565, on décrit l'extraction d'ecdystérone à partir de nymphes ou de chrysalides, destinée à la préparation de compositions cosmétiques.

Extraction à partir de plantes

La concentration en ecdystéroïdes, en particulier en ecdystérones, varie d'une variété à l'autre. Par exemple, la concentration en ecdystérone est de 0,025 % dans les graines d'Achyranthes aspera, de 0,35 % dans la plante Sesuvium portulacastrum, et de 1,2 % à 2,9 % dans Cyanotis arachnoïdea selon que l'on utilise la plante entière ou les racines. Les pourcentages sont des pourcentages en poids de matière sèche.

Parmi les plantes pouvant être utilisées pour l'extraction des ecdystéroïdes, en particulier de l'ecdystérone, on peut citer : Serratula sogdiana et Rhaponticum integrifolium, qui présentent l'avantage de pouvoir être cultivées (voir Horticultural Abs. 0C051-01546 en référence à Rastitel'nye Resursy, (1980), volume 16, n° 2, pages 193-198 (en Russe)).

On peut citer également les extractions à partir de :
- Serratula tinctoria (HUT-029 390) ;
- Serratula inermis (SU-1 146 050) ;
- Achyranthes fauriei, amaranthacée, (FR-1 525 385) ;
- Cyathula (FR-1 525 385 ) ;
- Ajuga decumbens (JP-46-014 665) ;
- Pfaffia iresinoides (Derwent 88-045806 ou JP-63 002 928) ;
- Pfaffia paniculata (Derwent 84-052423 ou JP-59-010 600);
- Polypodium vulgare (J. JIZBA et al. Tetrahedron Letters, (1967), page 1689, US 3 527 777, CS 131 136) ;
- Graine de Kaladana (DE-2 201 991) ;
- Ipomea petaloidea (DE-2 834 703) ;
- Silenes (HUT 029 390 et SU 924 051) ;
- Cyanotis arachnoidea (C.A. 89-176352 en référence à la publication de NIEN SCHUI LIN et al. ACTA CHIMICA SINICA 1978, volume 36, n° 2, pages 137-141 (en chinois)).

Ces sources d'ecdystéroïdes sont données sans caractère limitatif et ne sont pas exhaustives.

Certains extraits peuvent contenir, outre l'ecdystérone, également des dérivés acétylés (C.A. 89-176352).

On peut également obtenir les ecdystéroïdes selon l'invention par synthèse chimique (voir par exemple US-A-3 354 152, US-3 354 154, US-3 378 549, US-3 455 905, FR-1 494 371 ; US-3 440 241 ; FR-1 524 924 ; US-3 378 549 et FR-A-1 498 237).

Un exemple de procédé général d'extraction d'ecdystéroïde, de préférence d'ecdystérone, à partir de plantes est décrit dans Chem. Parm. Bull. (1969) 17 (2) 340-2 par S. Imai et al.

On fait macérer la plante fraîche dans 5 fois son poids de méthanol, on homogénéise et on filtre. On recommence une deuxième fois cette opération. On concentre les extraits, on ajoute de l'eau jusqu'à former une solution méthanol-eau à 30 %.

Cette solution est extraite à l'hexane. La fraction méthanolique à 30 % est concentrée à nouveau et extraite à l'acétate d'éthyle. La fraction aqueuse est extraite au n-butanol. L'extrait butanolique est alors concentré par évaporation, puis traité par chromatographie sur gel de silice par un mélange chloroforme-méthanol. On obtient ainsi l'ecdystérone, après recristallisation dans un mélange éthanol-acétate d'éthyle sous forme d'aiguilles incolores.

Par ailleurs, un certain nombre d'ecdystéroïdes sont disponibles dans le commerce. Par exemple l'ecdystérone, la 5-hydroxyecdystérone, la 2-désoxyecdystérone, l'ecdystérone 22-acétate, l'α-ecdysone et la 2-déoxy-α-ecdysone sont disponibles chez SIGMA sous les références SIGMA H 5142, SIGMA P 9531, SIGMA D 7775, SIGMA H 5267, SIGMA E 9004, SIGMA D 7900.

Les compositions selon l'invention précédemment décrites, contenant un ecdysteroïde, de préférence l'ecdystérone, ou ses dérivés, sous forme au moins en partie incorporée dans des phases lamellaires lipidiques hydratées ou dans des liposomes, trouvent de nombreuses applications.

Ainsi, selon un deuxième aspect de l'invention, l'invention concerne une composition cosmétique ou pharmaceutique, notamment dermatologique, caractérisée en ce qu'elle comprend une composition à base de phases lamellaires lipidiques hydratées ou des liposomes telle que précédemment définie, contenant au moins en partie au moins un ecdystéroïde ou un dérivé d'ecdystéroïde ou un extrait végétal ou animal contenant ledit ecdystéroïde ou un dérivé d'ecdystéroïde.

Ainsi, l'emploi en cosmétiques et pharmacie d'ecdystéroïde concerne notamment les produits anti-vieillissement, en particulier les produits anti-rides, les produits régénérants après soleil, les produits toniques pour les cheveux, les produits cicatrisants destinés à traiter notamment les plaies, les escarres,les ulcères veineux ou les brûlures, les produits anti-vergetures, les produits destinés à améliorer la résistance des parois des vaisseaux sanguins.

Egalement, en pharmacie, ces substances ont une action hypoglycémiante, hypolipidémiante et anti-athérosclérose, analgésique. L'ecdystérone est utilisée également pour la préparation de vaccins anti-parasitaires, notamment contre la bilharziose, pour réaliser des agents anti-viraux.

De préférence, la concentration en poids d'ecdystéroïde ou de ses dérivés dans lesdites compositions est comprise entre 0,001 et 5 %, de préférence encore entre 0,05 et 1 % par rapport au poids total de la composition.

Selon un troisième aspect, la présente invention concerne encore une composition de sériculture, caractérisée en ce qu'elle comprend une composition selon l'invention à base de phases lamellaires lipidiques hydratées ou de liposomes contenant au moins en partie au moins un ecdystéroïde ou un dérivé d'ecdystéroïdes ou un extrait végétal ou animal contenant ledit ecdystéroïde ou un dérivé d'ecdystéroïde.

La concentration en poids d'ecdystéroïde ou de ses dérivés dans cette composition de sériculture est la concentration classique et sera généralement comprise entre 0,0001 et 1 %, de préférence entre 0,0005 et 0,01 % par rapport au poids total de la composition.

L'emploi en sériculture d'ecdystérone est connu, comme facteur de métamorphose pour augmenter le rendement de la production de soie par le ver du Bombyx mori (voir FR-A-2 212 094, JP 46-014 665 ; JP 45-037 554).

Ainsi, les compositions selon l'invention peuvent être utilisées en pulvérisation sur les larves de ver à soie ou incorporées à leur nourriture.

Selon un quatrième aspect, l'invention concerne encore une composition phytosanitaire, caractérisée en ce qu'elle comprend une composition selon l'invention à base de phases lamellaires lipidiques hydratées ou de liposomes contenant au moins en partie au moins un ecdystéroïde ou un dérivé d'ecdystéroïde ou un extrait végétal ou animal contenant ledit ecdystéroïde ou un dérivé d'ecdystéroïde.

La concentration utilisée sera également la concentration habituelle, généralement comprise entre 0,0001 et 5 % en poids, de préférence entre 0,0005 et 1 % en poids par rapport au poids total de la composition.

L'utilisation des ecdysones pour la production d'insecticides ou de nématocides est connue par NL-A-67 10529, JP 47-003550; DE-2 201 991 ; FR-A-1 498 238, CS-131 136.

Dans toutes ces applications, l'incorporation au moins en partie d'ecdystéroïde, de préférence l'ecdystérone, dans des phases lamellaires lipidiques hydratées ou dans des liposomes apporte une augmentation inattendue de l'activité de ces substances par rapport à leur activité à l'état libre.

Ceci constitue donc un progrès technique déterminant tout à fait inattendu pour l'homme de l'art.

D'autres buts, caractéristiques et avantages de l'invention apparaîtront à la lumière de la description explicative qui va suivre faite en référence à plusieurs exemples de réalisation de l'invention donnés simplement à titre d'illustration et qui ne sauraient donc en aucune façon limiter la portée de l'invention.

Dans les exemples, les pourcentages sont donnés en poids, sauf indication contraire.

Exemple 1 selon l'invention

Préparation de liposomes contenant de l'ecdystérone.

On dissout 0,25 g d'ecdystérone du commerce, 4,5 g de lécithine de soja et 0,5 g de β-sitostérol dans un mélange dichlorométhane-méthanol 4 : 1. Cette solution est évaporée sous pression réduite (200 mm de mercure environ) dans un ballon rotatif porté à 45°C. Le film lipidique obtenu est repris par 119,75 g d'une solution aqueuse de 0,2 g/l de phosphate monopotassique et de 1,44 g/l de phosphate disodique sous agitation pendant 1 h.

On obtient ainsi une suspension de liposomes que l'on soumet à une homogénéisation aux ultrasons (15 min, 150 W, 4°C). Cette suspension est ensuite gélifiée en la mélangeant à poids égal avec un gel de Carbopol 940®, préparé de façon classique à 1,25 %.

On obtient ainsi 250 g d'une composition gélifiée à base de liposomes, dont la teneur en ecdystérone est de 0,1 %.

Exemples 2 et 3 selon l'invention

Préparation de phases lamellaires lipidiques hydratées contenant de l'ecdystérone, ainsi que l'ecdystérone 22-acétate

Dans 300 ml de dichlorométhane, on dissout les composés suivants :

| lécithine de soja | 36,0 g |
|---|---|
| α-tocophérol | 0,5 g |
| β-sitostérol | 4,0 g |
| ecdystérone* | 0,3 g |
| ecdystérone-22-acétate* | 0,2 g |

* du commerce

La solution obtenue est atomisée selon le procédé décrit dans le document EP-B1-0087 993 à 55°C, de façon à produire un mélange intime des constituants sous la forme d'une poudre fine. 20 g de la poudre lipidique atomisée ainsi obtenue sont ensuite dispersés dans 80 g d'une solution aqueuse, telle que décrite à l'exemple 1, au moyen d'un broyeur à rouleaux.

On obtient ainsi une phase lamellaire lipidique peu hydratée à 0,24 % environ d'ecdystéroïdes (exemple 2).

D'autre part, 15 g de poudre lipidique atomisée seront dispersés dans 485 g de la solution aqueuse précitée, sous agitation douce pendant 2 h. On obtient ainsi une suspension aqueuse de phases lamellaires très hydratées, ou liposomes, que l'on peut homogénéiser, par exemple au moyen d'un homogénéiseur sous pression comme décrit dans le document EP-B1-0 107 559. La suspension homogénéisée obtenue (exemple 3) contient des liposomes mesurant en moyenne 100 nm.

La teneur en ecdystéroïde est d'environ 0,04 % en poids de la suspension aqueuse.

Exemple 4 selon l'invention

Mise en évidence de l'activité des compositions selon l'invention sur la régénération de la structure cutanée.

L'activité sur la régénération de la structure cutanée des compositions selon l'invention a été mise en évidence chez le rat, par un test de rapidité de cicatrisation d'une lésion cutanée.

50 rats Hairless mâles, de 250 g environ sont répartis en 5 lots de 10 animaux dont 1 lot témoin sans traitement.

Une lésion est pratiquée sur chaque animal anesthésié au Pentobarbital sodique, à l'aide d'une sonde métallique de 13 mm de diamètre refroidie à l'azote liquide et appliquée 15 s sur la peau au niveau de la zone scapulaire.

Les animaux de 4 lots sont ensuite traités 5 jours sur 7, sur le site de la lésion, par 0,5 ml du produit à tester.

Les lots 1 a 4 sont traités respectivement par les produits A à D suivants :

Produit A :	suspension de liposomes selon l'invention selon l'exemple 1 à 0,1 % en ecdystérone
Produit B :	gel de Carbopol 940® à 0,625 % contenant 0,1 % en ecdystérone libre, comparatif
Produit C :	gel de carbopol 940® à 0,625 %, comparatif
Produit D :	suspension de liposomes préparés selon l'exemple 1, mais sans incorporation d'ecdystérone, comparatif.

Tous les jours, lors de l'administration, les animaux sont observés pour vérifier l'évolution de la croûte formée sur la lésion.

La disparition de cette croûte conditionne la fin de l'expérimentation et le sacrifice des animaux dont on prélève la peau pour l'étude histologique de la région traitée.

Le temps écoulé entre le début de traitement par les produits à tester et la disparition de la croûte est noté en jours.

Le nombre de jours de cicatrisation pour chaque lot est la moyenne arithmétique pour chaque animal traité. Chaque moyenne est comparée aux autres par un test de t de Student. Les résultats chiffrés figurent au tableau I.

TABLEAU I

| N° Lot | Produit | Moyenne (jours) Ecart-type | | Test de t | | | |
|---|---|---|---|---|---|---|---|
| 1 | A (invention) | 11,30 3,50 | A/B 3,35 S | A/C 2,05 (S) | A/D 5,23 HS | A/T 3,43 S | |
| 2 | B (comparatif) | 23,30 10,77 | | B/C 1,55 NS | B/D 1,44 NS | B/T 0,75 NS | |
| 3 | C (comparatif) | 16,80 7,73 | | | C/D 3,19 S | C/T 2,07 NS | |
| 4 | D (comparatif) | 30,30 10,93 | | | | D/T 0,47 NS | |
| 5 | Témoin | 27,60 14,60 | | | | | |

Ces résultats montrent très clairement que les liposomes contenant l'ecdystérone (produit A) présentent une activité significativement ou très significativement supérieure aux autres produits testés sur la régénération cutanée après lésion. En particulier, cette supériorité est très nette par rapport au gel de Carbopol contenant une concentration égale en ecdystérone (produit B).

Par ailleurs, l'étude histologique de la zone cicatricielle donne, pour les animaux ayant été traités par les produits A ou B, les résultats figurant au tableau II.

7

TABLEAU II

Répartition des animaux selon la réaction de cicatrisation

| N° Lot | Produit | Intensité de la réaction | | | |
|---|---|---|---|---|---|
| | | +++ | ++ | + | − |
| 1 | A (invention) | 0 | 3 | 5 | 2 |
| 2 | B (comparatif) | 1 | 1 | 1 | 7 |

Echelle de notation :

−    : forte réaction de cicatrisation

+    : réaction modérée de cicatrisation

++   : faible réaction de cicatrisation

+++  : structures normales.

La réaction de cicatrisation est caractérisée par un épiderme acanthosique avec une couche basale désorganisée, oedème à la jonction dermo-épidermique, derme avec présence de nombreux fibroblastes.

Ainsi, la restructuration de la peau après lésion est d'autant meilleure que la réaction de cicatrisation est faible.

Il apparaît clairement ici encore que le produit A (composition selon l'invention) présente une meilleure action de restructuration que le produit B (ecdystérone libre).

Exemple 5

Gel dermatologique selon l'invention

La composition selon l'exemple 1 peut être utilisée telle quelle comme composition dermatologique sous forme de gel.

Ce gel est appliqué, en cas notamment de brûlures, gerçures ou plaies, pendant une durée d'environ 8 à 15 jours et de préférence deux fois par jour.

Il est particulièrement utile pour accélérer la cicatrisation des plaies chirurgicales et notamment de la chirurgie esthétique.

Exemple 6

Composition cosmétique après solaire selon l'invention

La composition selon l'exemple 3 gélifiée, après mélange à 50-50 (en poids) avec un gel de Carbopol 940® à 2 %, peut être utilisée comme composition cosmétique réparatrice de l'élastose solaire et du vieillissement actinique.

Cette composition est, dans ce cas, appliquée de préférence deux fois par jour pendant trois semaines sur les zones à traiter.

Exemple 7

Crème cosmétique selon l'invention pour combattre les effets du vieillissement.

La composition de la crème est la suivante :

```
lécithine de soja                       1,95 g
β-sitostérol                            0,05 g
ecdystéroïdes                           0,2  g
(dont :
    ecdystérone*                  0,12 g
    ecdystérone-22-acétate*       0,08 g
excipients pour émulsion
    huile dans eau, +       qsp       100 g
    conservateurs + parfums
 * du commerce
```

On prépare séparément une suspension de liposomes selon l'exemple 3 d'une part, et une émulsion huile dans eau à base de squalane d'autre part.

On mélange ensuite ces deux préparations, par exemple au moyen d'un agitateur à hélice à vitesse modérée, à raison de 1 volume de suspension de liposomes pour 5 volumes d'émulsion.

La crème obtenue appliquée quotidiennement sur le visage permet à la peau de retrouver et de prolonger son aspect de jeunesse et son éclat.

**Revendications**

1. Composition à base de phases lamellaires lipidiques hydratées ou de liposomes, caractérisée en ce que lesdites phases lamellaires lipidiques hydratées ou lesdits liposomes contiennent au moins en partie au moins un ecdystéroïde ou un dérivé d'ecdystéroïde, ou un extrait végétal ou animal contenant ledit ecdystéroïde ou un dérivé dudit ecdystéroïde.

2. Composition selon la revendication 1, caractérisée en ce que l'ecdystéroïde précité est l'ecdystérone, ou un dérivé d'ecdystérone, notamment un dérivé acylé, hydroxylé ou désoxylé de celle-ci.

3. Composition selon la revendication 1 ou 2, caractérisée en ce que l'ecdystéroïde précité est un dérivé d'ecdystérone choisi parmi la β-ecdysone-2-acétate, la β-ecdysone-3-acétate, β-ecdysone-2,3-diacétate, la β-ecdysone-2,3,22-triacétate, la β-ecdysone-2,3,22,25-tétraacétate, la 5-hydroxyecdystérone et la 2-désoxyecdystérone.

4. Composition selon la revendication 1, caractérisée en ce que l'ecdystéroïde précité est l'β-ecdysone ou un dérivé de celle-ci, notamment un acétate.

5. Composition selon l'une des revendications 1 à 4, caractérisée en ce que l'extrait précité contenant l'ecdystéroïde, de préférence l'ecdystérone, est un extrait de Polypodium vulgare, de Ajuga decumbens ou de Cyanotis arachnoïdea.

6. Composition selon l'une des revendications 1 à 5, caractérisée en ce que l'ecdystéroïde précité ou un dérivé de celui-ci ou un extrait végétal ou animal le contenant est incorporé, au moins en partie, dans la phase lipidique des phases lamellaires lipidiques hydratées ou des liposomes.

7. Composition selon la revendication 6, caractérisée en ce que la concentration en ecdystéroïde est comprise entre 0,001 et 30 % en poids, et de préférence entre 0,01 et 10 % en poids de la phase lipidique.

8. Composition cosmétique ou pharmaceutique, notamment dermatologique à base de phases lamellaires lipidiques hydratées ou de liposomes, caractérisée en ce qu'elle comprend une composition à base de phases lamellaires lipidiques hydratées ou de liposomes selonl'une quelconque des revendications 1 à 7.

9. Composition selon la revendication 8, caractérisée en ce que la concentration en ecdystéroïde est comprise entre 0,001 et 5 % en poids, de préférence 0,05 % et 1 % en poids par rapport au poids total de la composition.

10. Composition pour la sériculture, caractérisée en ce qu'elle comprend une composition à base de phases lamellaires lipidiques hydratées ou de liposomes selon l'une quelconque des revendications 1 à 6, de préférence avec une concentration en poids d'ecdystéroïde comprise entre 0,0001 et 1 % en poids, de préférence entre 0,0005 et 0,01 % en poids par rapport au poids total de la composition.

11. Composition phytosanitaire, caractérisée en ce qu'elle comprend une composition à base de phases lamellaires lipidiques hydratées ou de liposomes selon l'une des revendications 1 à 6, de préférence avec une concentration en poids d'ecdystéroïde comprise entre 0,0001 et 5 %, encore mieux 0,005 et 1 %, par rapport au poids total de la composition.

12. Composition cosmétique pour la régénération de la structure cutanée, telle que composition cicatrisante ou composition anti-ride, caractérisée en ce qu'elle comprend une composition à base de phases lamellaires lipidiques hydratées ou de liposomes selon l'une quelconque des revendications 1 à 7 ou telle que définie à la revendication 8 ou 9.

13. Composition pharmaceutique pour la régénération de la structure cutanée, telle que composition cicatrisante ou composition anti-ride, caractérisée en ce qu'elle comprend une composition à base de phases lamellaires lipidiques hydratées ou de liposomes selon l'une quelconque des revendications 1 à 7 ou telle que définie à la revendication 8 ou 9.


## Patentansprüche

1. Zusammensetzung auf Basis von wasserhaltigen lamellaren Lipidphasen oder von Liposomen, dadurch gekennzeichnet, daß die wasserhaltigen lamellaren Lipidphasen bzw. die Liposome zumindest teilweise ein Ecdysteroid oder ein Ecdysteroidderivat oder einen ein solches Ecdysteroid oder Ecdysteroidderivat enthaltenden pflanzlichen oder tierischen Extrakt enthalten.

2. Zusammensetzung nach Anspruch 1, dadurch gekennzeichnet, daß das Ecdysteroid Ecdysteron oder ein Ecdysteronderivat, insbesondere ein acyliertes, hydroxyliertes oder desoxyliertes Derivat davon, ist.

3. Zusammensetzung nach Anspruch 1 oder 2, dadurch gekennzeichnet, daß das Ecdysteroid ein Ecdysteronderivat, ausgewählt aus β-Ecdyson-2-acetat, β-Ecdyson-3-acetat, β-Ecdyson-2,3-diacetat, β-Ecdyson-2,3,22-triacetat, β-Ecdyson-2,3,22,25-tetraacetat, 5-Hydroxyecdysteron und 2-Desoxyecdysteron, ist.

4. Zusammensetzung nach Anspruch 1, dadurch gekennzeichnet, daß das Ecdysteroid β-Ecdyson oder ein Derivat desselben, insbesondere ein Acetat, ist.

5. Zusammensetzung nach einem der Ansprüche 1 bis 4, dadurch gekennzeichnet, daß der das Ecdysteroid, vorzugsweise Ecdysteron, enthaltende Extrakt ein Extrakt aus Polypodium vulgare, Ajuga decumbens oder Cyanotis arachnoidea ist.

6. Zusammensetzung nach einem der Ansprüche 1 bis 5, dadurch gekennzeichnet, daß das Ecdysteroid oder Derivat desselben bzw, ein dieses enthaltender pflanzlicher oder tierischer Extrakt zumindest teilweise in der Lipidphase der wasserhaltigen lamellaren Lipidphasen oder der Liposome eingearbeitet ist.

7. Zusammensetzung nach Anspruch 6, dadurch gekennzeichnet, daß die Konzentration an Ecdysteroid zwischen 0,001 und 30 Gew.%, vorzugsweise zwischen 0,01 und 10 Gew.%, der Lipidphase beträgt.

8. Kosmetische oder pharmazeutische, insbesondere dermatologische, Zusammensetzung auf Basis von wasserhaltigen lamellaren Lipidphasen oder von Liposomen, dadurch gekennzeichnet, daß sie eine Zusammensetzung auf Basis von wasserhaltigen lamellaren Lipidphasen oder von Liposomen nach einem der Ansprüche 1 bis 7 aufweist.

9. Zusammensetzung nach Anspruch 8, dadurch gekennzeichnet, daß die Konzentration an Ecdysteroid zwischen 0,001 und 5 Gew.%, vorzugsweise zwischen 0,05 und 1 Gew.%, bezogen auf das Gesamtgewicht der Zusammensetzung, beträgt.

10. Zusammensetzung für die Seidenzucht, dadurch gekennzeichnet, daß sie eine Zusammensetzung auf Basis von wasserhaltigen lamellaren Lipidphasen oder von Liposomen nach einem der Ansprüche 1 bis 6, vorzugsweise mit einer Gewichtskonzentration an Ecdysteroid zwischen 0,0001 und 1 Gew.%, vorzugsweise zwischen 0,0005 und 0,01 Gew.%, bezogen auf das Gesamtgewicht der Zusammensetzung, aufweist.

11. Pflanzenschutzzusammensetzung, dadurch gekennzeichnet, daß sie eine Zusammensetzung auf Basis von wasserhaltigen lamellaren Lipidphasen oder von Liposomen nach einem der Ansprüche 1 bis 6, vorzugsweise mit einer Gewichtskonzentration an Ecdysteroid zwischen 0,0001 und 5 Gew.%, vorzugsweise zwischen 0,005 und 1 Gew.%, bezogen auf das Gesamtgewicht der Zusammensetzung, aufweist.

12. Kosmetische Zusammensetzung zur Regeneration der Hautstruktur, wie narbenbildungsfördernde Zusammensetzung oder Zusammensetzung gegen Falten, dadurch gekennzeichnet, daß sie eine Zusammensetzung auf Basis von wasserhaltigen lamellaren Lipidphasen oder von Liposomen nach einem der Ansprüche 1 bis 7 oder wie in Anspruch 8 oder 9 definiert aufweist.

13. Pharmazeutische Zusammensetzung zur Regeneration der Hautstruktur, wie narbenbildungsfördernde Zusammensetzung oder Zusammensetzung gegen Falten, dadurch gekennzeichnet, daß sie eine Zusammensetzung auf Basis von wasserhaltigen lamellaren Lipidphasen oder von Liposomen nach einem der Ansprüche 1 bis 7 oder wie in Anspruch 8 oder 9 definiert aufweist.

## Claims

1. Composition based on hydrated lipidic lamellar phases or liposomes, characterized in that said hydrated lipidic lamellar phases or said liposomes contain at least partly at least one ecdysteroid or one ecdysteroid derivative, or a plant extract or animal extract containing said ecdysteroid or a derivative of said ecdysteroid.

2. Composition according to claim 1, characterized in that said ecdysteroid is ecdysterone, or a derivative of ecdysterone, notably an acylated, hydroxylated or desoxylated derivative thereof.

3. Composition according to claim 1 or 2, characterized in that said ecdysteroid is an ecdyserone derivative selected from beta-ecdysone-2-acetate, beta-ecdysone-3-acetate, beta-ecdysone-2,3-diacetate, beta-ecdysone-2,3,22-triacetate, beta-ecdysone-2,3,22,25-tetraacetate, 5-hydroxyecdysterone and 2-desoxyecdysterone.

4. Composition according to claim 1, characterized in that said ecdysteroid is the beta-ecdysone or a derivative therefrom, notably an acetate.

5. Composition according to one of claims 1 to 4, characterized in that said extract containing ecdysteroid, preferably ecdysterone, is an extract of Polypodium vulgare, of Ajuga decumbens or of Cyanotis arachnoidea.

6. Composition according to one of claims 1 to 5, characterized in that said ecdysteroid or a derivative thereof or a plant or animal extract containing it is incorporated, at least partly, in the lipidic phase of the hydrated lipidic lamellar phases or of the liposomes.

7. Composition according to claim 6, characterized in that the ecdysteroid concentration is comprised between 0.001 and 30% by weight and preferably between 0.01 and 10% by weight of the lipidic phase.

8. Cosmetic or pharmaceutical composition, notably dermatological composition based on hydrated lipidic lamellar phases or liposomes, characterized in that it comprises a composition based on hydrated lipidic lamellar phases or liposomes according to any one of claims 1 to 7.

9. Composition according to claim 8, characterized in that the ecdysteroid concentration is comprised between 0.001 and 5% by weight, preferably 0.05% and 1% by weight with respect to the total weight of the composition.

10. Composition for sericulture, characterized in that it comprises a composition based on hydrated lipidic lamellar phases or liposomes according to any one of claims 1 to 6, preferably with a concentration by weight of ecdysteroid comprised between 0.0002 and 1% by weight preferably between 0.0005 and 0.01% by weight with respect to the total weight of the composition.

11. Phytosanitary composition, characterized in that it comprises a composition based on hydrated lipidic lamellar phases or liposomes according to one of claims 1 to 6, preferably with a concentration by weight of ecdysteroid comprised between 0.0001 and 5%, better still 0.005 to 1%, with respect to the total weight of the composition.

12. Cosmetic composition for regenerating the structure of the skin, such as healing composition or anti-wrinkle composition, characterized in that it comprises a composition based on hydrated lipidic lamellar phases or liposomes according to any one of claims 1 to 7 or such as defined in claim 8 or 9.

13. Pharmaceutical composition for regenerating the structure of the skin, such as healing composition or anti-wrinkle composition, characterized in that it comprises a composition based on hydrated lipidic lamellar phases or liposomes according to any one of claims 1 to 7 or such as defined in claim 8 or 9.